# EUROPEAN PATENT SPECIFICATION

(11) **EP 1 610 886 B1**
(45) Date of publication and mention of the grant of the patent: **18.11.2009**
(21) Application number: 04726535.0
(22) Date of filing: 08.04.2004
(51) Int. Cl.: B01D 59/30, A61K 51/12, C22B 59/00

(54) **METHOD OF OBTAINING GALLIUM-68 AND USE THEREOF AND DEVICE FOR CARRYING OUT SAID METHOD**
VERFAHREN ZUR GEWINNUNG VON GALLIUM-68 UND DESSEN VERWENDUNG UND HILFSMITTEL ZUR DURCHFÜHRUNG DIESES VERFAHRENS
PROCEDE PERMETTANT D'OBTENIR DU GALLIUM-68, UTILISATION DE CELUI-CI ET MOYEN PERMETTANT DE METTRE EN OEUVRE CE PROCEDE

(30) Priority: 11.04.2003 GB 0308407
(43) Date of publication of application: 04.01.2006
(73) Proprietor: GE Healthcare Limited, Little Chalfont Buckinghamshire HP7 9NA (GB)
(72) Inventor: VELIKYAN, Irina, Uppsala Imanet AB, S-751 85 Uppsala (SE); LANGSTROM, Bengt, Uppsala Imanet AB, S-751 85 Uppsala (SE); BEYER, Gerd J., Cuclotron Unite, CH-1211 Geneve 14 (CH)
(74) Representative: Hammett, Audrey Grace Campbell
(86) International application number: PCT/GB2004/001548
(87) International publication number: WO 2004/089517

(56) References cited:
- WO-A-03/059397
- GB-A- 2 056 471
- US-A- 4 330 507
- US-A- 6 071 490
- J. SCHUHMACHER, W. MAIER-BORST: "A new 68Ge/68Ga radioisotope generator system for production of 68Ga in dilute HCl" IINTERNATIONAL JOURNAL OF APPLIED RADIATION AND ISOTOPES, vol. 32, 1981, pages 31-36, XP001194822 GREAT BRITAIN cited in the application

## Description

The present invention relates to a method of obtaining ⁶⁸Ga from a ⁶⁸Ge/⁶⁸Ga generator and a method of producing ⁶⁸Ga-radiolabelled complexes using the obtained ⁶⁸Ga. The invention further relates to a kit, which could be used to obtain ⁶⁸Ga and a kit, which could be used for the production of ⁶⁸Ga-radiolabelled complexes.

PET imaging is a tomographic nuclear imaging technique that uses radioactive tracer molecules that emit positrons. When a positron meets an electron, the both are annihilated and the result is a release of energy in form of gamma rays, which are detected by the PET scanner. By employing natural substances that are used by the body as tracer molecules, PET does not only provide information about structures in the body but also information about the physiological function of the body or certain areas therein. A common tracer molecule is for instance 2-fluoro-2-deoxy-D-glucose (FDG), which is similar to naturally occurring glucose, with the addition of a ¹⁸F-atom. Gamma radiation produced from said positron-emitting fluorine is detected by the PET scanner and shows the metabolism of FDG in certain areas or tissues of the body, e.g. in the brain or the heart. The choice of tracer molecule depends on what is being scanned. Generally, a tracer is chosen that will accumulate in the area of interest, or be selectively taken up by a certain type of tissue, e.g. cancer cells. Scanning consists of either a dynamic series or a static image obtained after an interval during which the radioactive tracer molecule enters the biochemical process of interest. The scanner detects the spatial and temporal distribution of the tracer molecule. PET also is a quantitative imaging method allowing the measurement of regional concentrations of the radioactive tracer molecule.

Commonly used radionuclides in PET tracers are ¹¹C, ¹⁸F, ¹⁵O ¹³N or ⁷⁶Br. Recently, new PET tracers were produced that are based on radiolabelled metal complexes comprising a bifunctional chelating agent and a radiometal. Bifunctional chelating agents are chelating agents that coordinate to a metal ion and are linked to a targeting vector that will bind to a target site in the patient's body. Such a targeting vector may be a peptide that binds to a certain receptor, probably associated with a certain area in the body or with a certain disease. A targeting vector may also be oligonucleotide specific for e.g. an activated oncogene and thus aimed for tumour localisation. The advantage of such complexes is that the bifunctional chelating agents may be labelled with a variety of radiometals like, for instance, ⁶⁸Ga, ²¹³Bi or ⁸⁶Y. In this way, radiolabelled complexes with special properties may be "tailored" for certain applications.

⁶⁸Ga is of special interest for the production of Ga-radiolabelled metal complexes used as tracer molecules in PET imaging. ⁶⁸Ga is obtained from a ⁶⁸Ge/⁶⁸Ga generator, which means that no cyclotron is required. ⁶⁸Ga decays to 89% by positron emission of 2.92 MeV and its 68 min half life is sufficient to follow many biochemical processes *in vivo* without unnecessary radiation. With its oxidation state of +III, ⁶⁸Ga forms stable complexes with various types of chelating agents and ⁶⁸Ga tracers have been used for brain, renal, bone, blood pool, lung and tumour imaging.

However, the use of ⁶⁸Ga obtained from a ⁶⁸Ge/⁶⁸Ga generator for the production of ⁶⁸Ga-radiolabelled metal complexes used as PET tracer molecules may cause problems. ⁶⁸Ga eluate from a ⁶⁸Ge/⁶⁸Ga generator often contains ⁶⁸Ge which lead to low radionuclide purity of ⁶⁸Ga-radiolabelled metal complexes produced from the ⁶⁸Ga eluate. Furthermore, the eluate also contains so-called pseudo carriers, i.e. other metal cations like Fe³⁺, Al³⁺, Cu²⁺, Zn²⁺ and In³⁺, which compete with ⁶⁸Ga³⁺ in the subsequent complex formation reaction and eventually decrease the specific activity. A further disadvantage is, that ⁶⁸Ga eluate from a ⁶⁸Ge/⁶⁸Ga generator has a low ⁶⁸Ga concentration, i.e. in the picomolar to nanomolar range. As a consequence, the amount of chelating agent in a subsequent ⁶⁸Ga-radiolabelling reaction has to be high for the reaction to take place, which in turn leads to low specific activity. A high amount of chelating agent is especially problematic when ⁶⁸Ga-radiolabelled PET tracers that comprise a bifunctional chelating agent, i.e. a chelating agent linked to a targeting vector are produced as the patient will receive an unfavourable high amount of these tracers.

J. Schuhmacher et al. Int. J. appl. Radiat. Isotopes 32, 1981, 31-36 describe the use of a Bio-Rad AG 1 x 8 anion exchanger for treating the 4.5 N HCl ⁶⁸Ga eluate obtained from a ⁶⁸Ge/⁶⁸Ga generator in order to decrease the amount of ⁶⁸Ge present in the eluate. 4 mL water was used to eluate the anion exchanger. A disadvantage of this method is the high volume of water, which is necessary to eluate the ⁶⁸Ga from the anion exchanger. In order to use this eluate for the production of ⁶⁸Ga-radiolabelled PET tracers that comprise a bifunctional chelating agent, the eluate needs to be further concentrated, e.g. by evaporation which in turn leads to a decrease of ⁶⁸Ga activity due to the short half-life of this radionuclide. US 6,071, 490 describes certain ⁶⁸Ga chelates and methods for preparing them. WO 03/059397 describes a method and compositions for labelling a targeting agent with ⁶⁸Ga
There is a need for a method of obtaining ⁶⁸Ga from a ⁶⁸Ge/⁶⁸Ga generator in such a way, that ⁶⁸Ga may be used for the production of ⁶⁸Ga-radiolabelled metal complexes, especially for the production of ⁶⁸Ga-radiolabelled PET tracers that comprise a bifunctional chelating agent with a high specific radioactivity.

It has now been found that the use of anion exchangers comprising HCO₃⁻ as counterions is particularly suitable for the purification and concentration of ⁶⁸Ga from a ⁶⁸Ge/⁶⁸Ga generator. Not only the amount of ⁶⁸Ge present in the eluate could be reduced but also the amount of pseudo carriers. Furthermore, the concentration of ⁶⁸Ga³⁺ could be increased up to a nanomolar to micromolar level. Hence, it was possible to reduce the amount of chelating agent in a subsequent complex formation reaction, which considerably increased the specific radioactivity. This result is important for the production of ⁶⁸Ga-radiolabelled PET tracers that comprise a bifunctional chelating agent; i.e. a chelating agent linked to a targeting vector, as the increase in specific radioactivity enables the reduction in amount of such tracers when used in a patient.

The invention thus provides a method of obtaining ⁶⁸Ga by contacting the eluate from a ⁶⁸Ge/⁶⁸Ga generator with an anion exchanger comprising HCO₃⁻ as counterions, and eluting ⁶⁸Ga from said anion exchanger.

⁶⁸Ge/⁶⁸Ga generators are known in the art, see for instance C. Loc'h et al, J. Nucl. Med. 21, 1980, 171-173 or J. Schuhmacher et al. Int. J. appl. Radiat. Isotopes 32, 1981, 31-36. ⁶⁸Ge may be obtained by cyclotron production by irradiation of, for instance Ga₂(SO₄)₃ with 20 MeV protons. It is also commercially available, e.g. as ⁶⁸Ge in 0.5 M HCl. Generally, ⁶⁸Ge is loaded onto a column consisting of organic resin or an inorganic metal oxide like tin dioxide, aluminium dioxide or titanium dioxide. ⁶⁸Ga is eluted from the column with aqueous HCl yielding ⁶⁸GaCl₃. Thus, ⁶⁸Ga is in the form of ⁶⁸Ga³⁺, which could be used in the synthesis of ⁶⁸Ga-radiolabelled complexes, e.g. for the production of ⁶⁸Ga-radiolabelled PET tracers.

Suitable columns for ⁶⁸Ge/⁶⁸Ga generators consist of inorganic oxides like aluminium dioxide, titanium dioxide or tin dioxide or organic resins like resins comprising phenolic hydroxyl groups (US-A-4264468) or pyrogallol (J. Schuhmacher et al., Int. J. appl. Radiat. Isotopes 32, 1981, 31-36). In a preferred embodiment, a ⁶⁸Ge/⁶⁸Ga generator comprising a column comprising titanium dioxide is used in the method according to the invention.

The concentration of the aqueous HCl used to elute ⁶⁸Ga from the ⁶⁸Ge/⁶⁸Ga generator column depends on the column material. Suitably 0.05 to 5 M HCl is used for the elution of ⁶⁸Ga. In a preferred embodiment, the eluate is obtained from a ⁶⁸Ge/⁶⁸Ga generator comprising a column comprising titanium dioxide and ⁶⁸Ga is eluted using 0.05 to 0.1 M HCl, preferably about 0.1 M HCl.

In a preferred embodiment of the method according to the invention, a strong anion exchanger comprising HCO₃⁻ as counterions, preferably a strong anion exchanger comprising HCO₃⁻ as counterions, is used. In a further preferred embodiment, this anion exchanger comprises quaternary amine functional groups. In another further preferred embodiment, this anion exchanger is a strong anion exchange resin based on polystyrene-divinylbenzene. In a particularly preferred embodiment, the anion exchanger used in the method according to the invention is a strong anion exchange resin comprising HCO₃⁻ as counterions, quaternary amine functional groups and the resin is based on polystyrene-divinylbenzene.

Suitably, water is used to elute the ⁶⁸Ga from the anion exchanger in the method according to the invention.

The ⁶⁸Ga obtained according to the method of the invention is preferably used for the production of ⁶⁸Ga-radiolabelled complexes, preferably for the production of ⁶⁸Ga-radiolabelled PET tracers that comprise a bifunctional chelating agent, i.e. a chelating agent linked to a targeting vector.

Thus, another aspect of the invention is a method for producing a ⁶⁸Ga-radiolabelled complex by
a) obtaining ⁶⁸Ga by contacting the eluate from a ⁶⁸Ge/⁶⁸Ga generator with an anion exchanger comprising HCO₃⁻ as counterions and eluting ⁶⁸Ga³⁺ from said anion exchanger, and
b) reacting the ⁶⁸Ga with a chelating agent.

Preferred chelating agents for use in the method of the invention are those which present ⁶⁸Ga in a physiologically tolerable form. Further preferred chelating agents are those that form complexes with ⁶⁸Ga that are stable for the time needed for diagnostic investigations using the radiolabelled complexes.

Suitable chelating agents are, for instance, polyaminopolyacid chelating agents like DTPA, EDTA, DTPA-BMA, DOA3, DOTA, HP-DOA3, TMT or DPDP. Those chelating agents are well known for radiopharmaceuticals and radiodiagnosticals. Their use and synthesis are described in, for example, US-A-4647447, US-A-5362 475, US-A-5534241, US-A-5358704, US-A-5198208, US-A-4963344, EP-A-230893, EP-A-130934, EP-A-606683, EP-A-438206, EP-A-434345, WO-A-97/00087, WO-A-96/40274, WO-A-96/30377, WO-A-96/28420, WO-A- 96/16678, WO-A-96/11023, WO-A-95/32741, WO-A-95/27705, WO-A-95/26754, WO-A-95/28967, WO-A-95/28392, WO-A-95/24225, WO-A-95/17920, WO-A-95/15319, WO-A-95/09848, WO-A-94/27644, WO-A-94/22368, WO-A-94/08624, WO-A-93/16375, WO-A-93/06868, WO-A-92/11232, WO-A-92/09884, WO-A-92/08707, WO-A-91/15467, WO-A-91/10669, WO-A-91/10645, WO-A-91/07191, WO-A-91/05762, WO-A-90/12050, WO-A-90/03804, WO-A-89/00052, WO-A-89/00557, WO-A-88/01178, WO-A-86/02841 and WO-A-86/02005.

Suitable chelating agents include macrocyclic chelating agents, e.g. porphyrin-like molecules and pentaaza-macrocycles as described by Zhang et al., Inorg. Chem. 37(5), 1998, 956-963, phthalocyanines, crown ethers, e.g. nitrogen crown ethers such as the sepulchrates, cryptates etc., hemin (protoporphyrin IX chloride), heme and chelating agents having a square-planar symmetry.

Macrocyclic chelating agents are preferably used in the method of the invention. In a preferred embodiment, these macrocyclic chelating agents comprise at least one hard donor atom such as oxygen and/or nitrogen like in polyaza- and polyoxomacrocycles. Preferred examples of polyazamacrocyclic chelating agents include DOTA, TRITA, TETA and HETA with DOTA being particularly preferred.

Particularly preferred macrocyclic chelating agents comprise functional groups such as carboxyl groups or amine groups which are not essential for coordinating to Ga³⁺ and thus may be used to couple other molecules, e.g. targeting vectors, to the chelating agent. Examples of such macrocyclic chelating agents comprising functional groups are DOTA, TRITA or HETA.

In a further preferred embodiment, bifunctional chelating agents are used in the method according to the invention. "Bifunctional chelating agent" in the context of the invention means chelating agents that are linked to a targeting vector. Suitable targeting vectors for bifunctional chelating agents useful in the method according to the invention are chemical or biological moieties, which bind to target sites in a patient's body, when the ⁶⁸Ga-radiolabelled complexes comprising said targeting vectors have been administered to the patient's body. Suitable targeting vectors for bifunctional chelating agents useful in the method according to the invention are proteins, glycoproteins, lipoproteins, polypeptides like antibodies or antibody fragments, glycopolypeptides, lipopolypeptides, peptides, like RGD binding peptides, glycopeptides, lipopeptides, carbohydrates, nucleic acids, e.g. DNA, RNA, oligonucleotides like antisense oligonucleotides or a part, a fragment, a derivative or a complex of the aforesaid compounds, or any other chemical compound of interest like relatively small organic molecules, particularly small organic molecules of less than 2000 Da.

In a particularly preferred embodiment, macrocyclic bifunctional chelating agents are used in the method according to the invention. Preferred macrocyclic bifunctional chelating agents comprise DOTA, TRITA or HETA linked to a targeting vector, preferably to a targeting vector selected from the group consisting of proteins, glycoproteins, lipoproteins, polypeptides, glycopolypeptides, lipopolypeptides, peptides, glycopeptides, lipopeptides carbohydrates, nucleic acids, oligonucleotides or a part, a fragment, a derivative or a complex of the aforesaid compounds and small organic molecules; particularly preferably to a targeting vector selected from the group consisting of peptides and oligonucleotides.

The targeting vector can be linked to the chelating agent via a linker group or via a spacer molecule. Examples of linker groups are disulfides, ester or amides, examples of spacer molecules are chain-like molecules, e.g. lysin or hexylamine or short peptide-based spacers. In a preferred embodiment, the linkage between the targeting vector and the chelating agent part of radiolabelled gallium complex is as such that the targeting vector can interact with its target in the body without being blocked or hindered by the presence of the radiolabelled gallium complex.

A preferred aspect of the invention is a method for producing a ⁶⁸Ga-radiolabelled complex by
c) obtaining ⁶⁸Ga by contacting the eluate from a ⁶⁸Ge/⁶⁸Ga generator with an anion exchanger comprising HCO₃⁻ as counterions and eluting ⁶⁸Ga from said anion exchanger, and
d) reacting the ⁶⁸Ga with a chelating agent, wherein the reaction is carried out using microwave activation.

It has been found that the use of microwave activation substantially improves the efficiency and reproducibility of the ⁶⁸Ga-chelating agent complex formation. Due to microwave activation, chemical reaction times could be shortened substantially; i.e. the reaction is completed within 2 min and less. This is a clear improvement as a 10 minutes shortage of the reaction time saves about 10% of the ⁶⁸Ga activity. Furthermore, microwave activation also leads to fewer side reactions and to an increased radiochemical yield, which is due to increased selectivity.

Suitably, a microwave oven, preferably a monomodal microwave oven is used to carry out microwave activation. Suitably microwave activation is carried out at 80 to 120 W, preferably at 90 to 110 W, particularly preferably at about 100 W. Suitable microwave activation times range from 20 s to 2 min, preferably from 30 s to 90 s, particularly preferably from 45 s to 60 s.

A temperature control of the reaction is advisable when temperature sensitive chelating agents, like for instance bifunctional chelating agents comprising peptides or proteins as targeting vectors, are employed in the method according to the invention. Duration of the microwave activation should be adjusted in such a way, that the temperature of the reaction mixture does not lead to the decomposition of the chelating agent and/or the targeting vector. If chelating agents used in the method according to the invention comprise peptides or proteins, higher temperatures applied for a shorter time are generally more favourable than lower temperatures applied for a longer time period.

Microwave activation can be carried out continuously or in several microwave activation cycles during the course of the reaction.

Another aspect of the invention is a kit for obtaining ⁶⁸Ga from a ⁶⁸Ge/⁶⁸Ga generator, which comprises a generator column and a second column that comprises an anion exchanger comprising HCO₃⁻ as counterions.

In a preferred embodiment, the kit further comprises means to couple the columns in series and/or aqueous HCl to elute the ⁶⁸Ga from the generator column and/or water to elute the ⁶⁸Ga from the anion exchanger column. The HCl and the water are preferably aseptically and in a hermetically sealed container.

In another preferred embodiment, the kit according to the invention further comprises a chelating agent, preferably a bifunctional chelating agent, i.e. a chelating agent linked to a targeting vector.

### Examples

### Example 1:

0.1 M HCl (5-6 mL) was used to elute ⁶⁸Ga from a ⁶⁸Ge/⁶⁸Ga-generator with a titanium dioxide column. The eluate was acidified with HCl and applied to a cartridge containing a strong basic anion exchange resin based on polystyrene-divinylbenzene comprising HCO₃⁻ as counterions and quaternary amine functional groups (SPE cartridge Chromafix 30-PS-HCO₃, Macharey-Nagel, Germany). Over 99% of the ⁶⁸Ga activity was retained on the resin and then eluted with 200 µl of H₂O.

### Comparative Example 1a

The eluate obtained according to Example 1 was applied to cartridges containing a strong basic anion exchange resin comprising Cl⁻ as counterions and quaternary amine functional groups (SAX SPEC, 50 mg, 1 mL, Isolute, UK and SAX SPEC, 15 mg, 3 mL, NTK kemi, USA). Both anion exchangers did not show any ⁶⁸Ga activity retention.

### Comparative Example 1b

The Cl⁻ counterions of the cartridges used in Comparative Example 1a were exchanged with OH⁻ counterions and the Example was carried out as described in Example 1a. The retention of ⁶⁸Ga activity was 10 - 20 %.

### Example 2:

### Comparative study of ⁶⁸Ga-radiolabelling of DOTA-D-Phe¹-Tyr³ - Octreotide (DOTA-TOC).

⁶⁸Ga obtained according to the method of the invention and ⁶⁸Ga obtained from a ⁶⁸Ge/⁶⁸Ga-generator without further anion exchanger-treatment are used for ⁶⁸Ga-radiolabelling of DOTA-D-Phe¹-Tyr³ - Octreotide (DOTA-TOC).

### 2a) ⁶⁸Ga - radiolabelling of DOTA-TOC

Sodium acetate was added to the eluate from the ⁶⁸Ge/⁶⁸Ga-generator (36 mg to 1 mL) to adjust a pH of approximately 5.5 and the mixture was vortexed well. DOTA-TOC (20 nmol) was added and the mixture was heated at 96 °C for 25 min. The reaction mixture was cooled to room temperature and applied to a C-18 SPE-column (HyperSEP S C18), which was then washed with 2 mL H₂O and the product was eluted with ethanol: water 50:50 (1 mL).

The reaction mixture and the product were analysed by HPLC using Vydac RP and Fast Desalting HR 10/10 FPLC gel filtration columns. Specific radioactivity (the amount of radioactivity per unit mass of the peptide) was 1.5 MBq/nmol. Electrospray ionization mass spectrometry, ESI-MS, was performed on Fisons Platform (Micromass, Manchester, UK), using positive mode scanning and detecting [M+2H]²⁺. DOTATOC was detected at m/z =711.26 and authentic Ga-DOTATOC was detected at m/z = 746.0 (calculated m/z = 746.5).

### 2b) ⁶⁸Ga - radiolabelling of DOTA-TOC using ⁶⁸Ga obtained from Example 1

Sodium acetate was added to the 200 µl of ⁶⁸Ga obtained from Example 1 to adjust a pH of approximately 5.5. The complex formation reaction was carried out as described in Example 2a) using 10 nmol DOTATOC.

The reaction mixture and the product were analysed by HPLC using Vydac RP and Fast Desalting HR 10/10 FPLC gel filtration columns. Specific radioactivity was 5 MBq/nmol. Electrospray ionization mass spectrometry, ESI-MS, was performed on Fisons Platform (Micromass, Manchester, UK), using positive mode scanning and detecting [M+2H]²⁺. DOTATOC was detected at m/z =711.26 and authentic Ga-DOTATOC was detected at m/z = 746.0 (calculated m/z = 746.5).

### 2c) Results

In the case of the use of ⁶⁸Ga obtained by the method of the invention, it was possible to decrease the amount of DOTATOC needed for the synthesis of the radiolabelled complex and thus increase the specific radioactivity more than 3 times.

## Claims

1. Method of obtaining ⁶⁸Ga by contacting the eluate from a ⁶⁸Ge/⁶⁸Ga generator with an anion exchanger comprising HCO₃⁻ as counterions and eluting ⁶⁸Ga from said anion exchanger.

2. Method according to claim 1 wherein the ⁶⁸Ge/⁶⁸Ga generator comprises a column comprising titanium dioxide.

3. Method according to claim 1 wherein 0.05 to 5 M HCl is used to elute ⁶⁸Ga from the ⁶⁸Ge/⁶⁸Ga generator.

4. Method according to claim 2 wherein 0.05 to 0.1 M HCl is used to elute ⁶⁸Ga from the ⁶⁸Ge/⁶⁸Ga generator.

5. Method according to claims 1 to 4 wherein water is used to elute ⁶⁸Ga from the anion exchanger.

6. Method according to claims 1 to 5 wherein the anion exchanger is a strong anion exchanger comprising quaternary amine functional groups.

7. Method according to claims 1 to 6 wherein the anion exchanger is a strong anion exchange resin based on polystyrene-divinylbenzene.

8. Method according to any one of claims 1 to 7 comprising the further step of producing a ⁶⁸Ga-radiolabelled complex by reacting the ⁶⁸Ga so obtained with a chelating agent.

9. Method according to claim 8 wherein the chelating agent is a macrocyclic chelating agent.

10. Method according to claims 8 to 9 wherein the chelating agent comprises hard donor atoms, preferably O and N.

11. Method according to claims 8 to 10 wherein the chelating agent is a bifunctional chelating agent

12. Method according to claim 11 wherein the chelating agent is a bifunctional chelating agent comprising a targeting vector selected from the group consisting of proteins, glycoproteins, lipoproteins, polypeptides, glycopolypeptides, lipopolypeptides, peptides, glycopeptides, lipopeptides, carbohydrates, nucleic acids, oligonucleotides or a part, a fragment, a derivative or a complex of the aforesaid compounds and small organic molecules.

13. Method according to claims 8 to 12 wherein the reaction is carried out using microwave activation.

14. Method according to claims 8 to 13 for the production of ⁶⁸Ga-radiolabelled PET tracers.

15. Kit for the preparation of ⁶⁸Ga from a ⁶⁸Ge/⁶⁸Ga generator, which comprises a generator column and a second column that comprises an anion exchanger comprising HCO₃⁻ as counterions.

16. Kit according to claim 15 further comprising means to couple the columns in series.

17. Kit according to claims 15 to 16 further comprising aqueous HCl to elute the ⁶⁸Ga from the generator column and/or water to elute the ⁶⁸Ga from the anion exchanger column, preferably, the HCl and the water being aseptically and in a hermetically sealed container.

18. Kit according to claims 15 to 17 further comprising a chelating agent, preferably a bifunctional chelating agent.

19. Use of a kit according to claim 18 for the production of ⁶⁸Ga-radiolabelled PET tracers.

## Patentansprüche

1. Verfahren zur Gewinnung von ⁶³Ga durch In-Kontakt-Bringen des Eluats aus einem ⁶⁸Ge/⁶⁸Ga-Generator mit einem Anionenaustauscher, der HCO₃⁻ als Gegenionen umfasst, und Eluieren von ⁶⁸Ga aus dem Anionenaustauscher.

2. Verfahren nach Anspruch 1, wobei der ⁶⁸Ge/⁶⁸Ga-Generator eine Titandioxid umfassende Säule umfasst.

3. Verfahren nach Anspruch 1, wobei 0,05 bis 5 M HCl verwendet werden, um ⁶⁸Ga aus dem ⁶⁸Ge/⁶⁸Ga-Generator zu eluieren.

4. Verfahren nach Anspruch 2, wobei 0,05 bis 0,1 M HCl verwendet werden, um ⁶⁸Ga aus dem ⁶⁸Ge/⁶⁸Ga-Generator zu eluieren.

5. Verfahren nach Anspruch 1 bis 4, wobei Wasser verwendet wird, um ⁶⁸Ga aus dem Anionenaustauscher zu eluieren.

6. Verfahren nach Anspruch 1 bis 5, wobei der Anionenaustauscher ein starker Anionenaustauscher ist, der quaternäres Amin als funktionelle Gruppen umfasst.

7. Verfahren nach Anspruch 1 bis 6, wobei der Anionenaustauscher ein starkes Anionenaustauschharz ist, das auf Polystyrol-Divinylbenzol basiert.

8. Verfahren nach einem der Ansprüche 1 bis 7, umfassend den weiteren Schritt des Herstellens eines ⁶⁸Ga-radioaktiv-markierten Komplexes durch Umsetzen des so gewonnenen ⁶⁸Ga mit einem Chelatbildner.

9. Verfahren nach Anspruch 8, wobei der Chelatbildner ein makrocyclischer Chelatbildner ist.

10. Verfahren nach Anspruch 8 bis 9, wobei der Chelatbildner harte Donatoratome umfasst, bevorzugt O und N.

11. Verfahren nach Anspruch 8 bis 10, wobei der Chelatbildner ein bifunktioneller Chelatbildner ist.

12. Verfahren nach Anspruch 11, wobei der Chelatbildner ein bifunktioneller Chelatbildner ist, der einen Targeting-Vektor umfasst, der aus jener Gruppe ausgewählt wird, die besteht aus Proteinen, Glykoproteinen, Lipoproteinen, Polypeptiden, Glykopolypeptiden, Lipopolypeptiden, Peptiden, Glykopeptiden, Lipopeptiden, Kohlenhydraten, Nukleinsäuren, Oligonukleotiden oder einem Teil, einem Fragment, einem Derivat oder einem Komplex der obengenannten Verbindungen und kleinen organischen Molekülen.

13. Verfahren nach Anspruch 8 bis 12, wobei die Umsetzung mittels Mikrowellenaktivierung durchgeführt wird.

14. Verfahren nach Anspruch 8 bis 13 zur Herstellung von ⁶⁸Ga-radioaktivmarkierten PET-Tracern.

15. Kit zur Herstellung von ⁶⁸Ga aus einem ⁶⁸Ge/⁶⁸Ga-Generator, der eine Generatorsäule und eine zweite Säule umfasst, die einen Anionenaustauscher umfasst, der HCO₃⁻ als Gegenionen umfasst.

16. Kit nach Anspruch 15, weiterhin umfassend Mittel, um die Säulen in Reihe zu koppeln.

17. Kit nach Anspruch 15 bis 16, weiterhin umfassend wässriges HCl, um das ⁶⁸Ga aus der Generatorsäule zu eluieren, und/oder Wasser, um das ⁶⁸Ga aus der Anionenaustauschersäule zu eluieren, wobei das HCl und das Wasser bevorzugt aseptisch und in einem hermetisch verschlossenen Behälter sind.

18. Kit nach Anspruch 15 bis 17, weiterhin umfassend einen Chelatbildner, bevorzugt einen bifunktionellen Chelatbildner.

19. Verwendung eines Kits nach Anspruch 18 zur Herstellung von ⁶⁸Ga-radioaktivmarkierten PET-Tracern.

## Revendications

1. Procédé d'obtention de ⁶⁸Ga en mettant en contact le produit élué provenant d'un générateur de ⁶⁸Ge/⁶⁸Ga avec un échangeur d'anion comprenant du HCO₃⁻ en tant que contre-ions et en éluant le ⁶⁸Ga par rapport audit échangeur d'anion.

2. Procédé selon la revendication 1 dans lequel le générateur de ⁶⁸Ge/⁶⁸Ga comprend une colonne comprenant du dioxyde de titane.

3. Procédé selon la revendication 1 dans lequel de 0,05 à 5 M de HCl sont utilisés afin d'assurer l'élution du ⁶⁸Ga par rapport au générateur ⁶⁸Ge/⁶⁸Ga.

4. Procédé selon la revendication 2, dans lequel de 0,05 à 0,1 M de HCl est utilisé afin d'assurer l'élution du ⁶⁸Ga par rapport au générateur ⁶⁸Ge/⁶⁸Ga.

5. Procédé selon les revendications 1 à 4, dans lequel de l'eau est utilisée afin d'assurer l'élution du ⁶⁸Ga par rapport à l'échangeur d'anion.

6. Procédé selon les revendications 1 à 5, dans lequel l'échangeur d'anion est un échangeur d'anion fort comprenant des groupes fonctionnels amines quaternaires.

7. Procédé selon les revendications 1 à 6, dans lequel l'échangeur d'anion est une résine d'échange anionique forte basée sur le polystyrène-divinylbenzène.

8. Procédé selon l'une quelconque des revendications 1 à 7, comprenant l'étape supplémentaire de production d'un complexe marqué par ⁶⁸Ga en faisant réagir le ⁶⁸Ga ainsi obtenu avec un agent de chélation.

9. Procédé selon la revendication 8, dans lequel l'agent de chélation est un agent de chélation macrocyclique.

10. Procédé selon les revendications 8 et 9, dans lequel l'agent de chélation comprend des atomes donneurs durs, de préférence, O et N.

11. Procédé selon les revendications 8 à 10, dans lequel l'agent de chélation est un agent de chélation bifonctionnel.

12. Procédé selon la revendication 11, dans lequel l'agent de chélation est un agent de chélation bifonctionnel comprenant un vecteur de ciblage sélectionné dans le groupe constitué par les protéines, les glycoprotéines, les lipoprotéines, les polypeptides, les glycopolypeptides, les lipopolypeptides, les peptides, les glycopeptides, les lipopeptides, les hydrates de carbone, les acides nucléiques, les oligonucléotides ou une partie, un fragment, un dérivé ou un complexe des composés mentionnés ci-dessus et de petites molécules organiques.

13. Procédé selon les revendications 8 à 12 dans lequel la réaction est mise en oeuvre en utilisant une activation par micro-onde.

14. Procédé selon les revendications 8 à 13 afin d'assurer la production de traceurs de tomographie PET marqués par ⁶⁸Ga.

15. Kit de préparation de ⁶⁸Ga à partir d'un générateur de ⁶⁸Ge/⁶⁸Ga, qui comprend une colonne de générateur et une deuxième colonne qui comprend un échangeur d'anion comprenant du HCO₃⁻ en tant que contre-ions.

16. Kit selon la revendication 15, comprenant, en outre, des moyens destinés à coupler les colonnes en série.

17. Kit selon les revendications 15 et 16, comprenant, en outre, du HCl aqueux afin d'assurer l'élution du ⁶⁸Ga à partir de la colonne de générateur et/ou de l'eau afin d'assurer l'élution du ⁶⁸Ga à partir de la colonne d'échangeur d'anion, de préférence, le HCl et l'eau étant dans un conteneur scellé de manière aseptique et étanche.

18. Kit selon les revendications 15 à 17, comprenant, en outre, un agent de chélation, de préférence, un agent de chélation bifonctionnel.

19. Utilisation d'un kit selon la revendication 18 afin d'assurer la production de traceurs de PET marqués par ⁶⁸Ga.
